(19) Europäisches Patentamt
European Patent Office
Office européen des brevets

(11) Veröffentlichungsnummer: **0 011 877**
**B1**

(12) # EUROPÄISCHE PATENTSCHRIFT

(45) Veröffentlichungstag der Patentschrift: **20.05.81**

(21) Anmeldenummer: **79104850.7**

(22) Anmeldetag: **03.12.79**

(51) Int. Cl.³: **C 07 C 49/417**, C 07 C 49/617, C 07 C 45/61, C 11 B 9/00, A 61 K 7/46

(54) **Neue Caranderivate (I), Verfahren zu deren Herstellung, Verwendung von I als Riechstoffe sowie Riechstoffkompositionen mit einem Gehalt an I.**

(30) Priorität: **05.12.78 CH 12390/78**

(43) Veröffentlichungstag der Anmeldung: **11.06.80 Patentblatt 80/12**

(45) Bekanntmachung des Hinweises auf die Patenterteilung: **20.05.81 Patentblatt 81/20**

(84) Benannte Vertragsstaaten: **CH DE FR GB NL**

(56) Entgegenhaltungen:
**US - A - 3 560 571**
**US - A - 3 686 097**

(73) Patentinhaber: **L. Givaudan & Cie Société Anonyme**
**Patentdienst Postfach**
**CH-4002 Basel (CH)**

(72) Erfinder: **Lamparsky, Dietmar, Dr.**
**Sonnhalde 8**
**CH-8602 Wangen (CH)**

(74) Vertreter: **Lederer, Franz, Dr. et al.**
**Patentanwälte Dr. Franz Lederer Reiner F. Meyer**
**Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 011 877 B1

Neue Caranderivate (I), Verfahren zu deren Herstellung, Verwendung von I als Riechstoffe sowie Riechstoffkompositionen mit einem Gehalt an I.

Die Erfindung betrifft neue Riechstoffe. Es handelt sich dabei um die Verbindungen der Formel

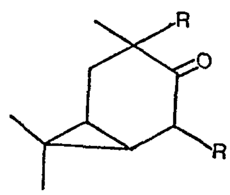

I

worin der eine Rest R für 3-Methyl-2-butenyl (Prenyl-) und der andere für Wasserstoff steht.

Die Formel soll demgemäss das 3,7,7-Trimethyl-3-[3'-methyl-2'-butenyl]-bicyclo[4.1.0]-heptan-4-on Ia und das 3,7,7-Trimethyl-5-[3'-methyl-2'-butenyl]-bicyclo[4.1.0]-heptan-4-on Ib

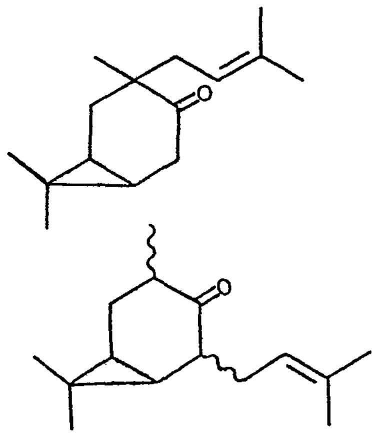

Ia

Ib

als auch alle durch die Substituenten am Sechsring möglichen Diastereoisomeren von Ia und Ib umfassen.

Die Erfindung betrifft ferner ein Verfahren zur Herstellung der Verbindungen der Formel I.

Dieses Verfahren ist dadurch gekennzeichnet, dass man 4-Caranon, also die Verbindung der Formel

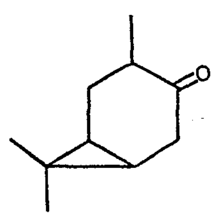

II

mit einem Prenylhalogenid umsetzt.

Als ausgangsmaterial wird vorzugsweise cis-4-Caranon eingesetzt.

Als Prenylhalogenide kommen alle Halogenide in Frage, doch wird vorzugsweise das Chlorid verwendet.

Die Umsetzung erfolgt vorzugsweise in Gegenwart einer starken anorganischen Base, wie z.B. einem Alkalimetallhydroxid, z.B. KOH, einem Erdalkalihydroxid, z.B. $Ca(OH)_2$, einem Alkalimetallamid, z.B. $NaNH_2$, oder einem Alkalimetallhydrid, z.B. $NaH_2$, oder aber auch einer organischen Base, wie z.B. Kalium-tert.butoxid.

Man kann mit oder ohne Zusatz eines Lösungsmittels arbeiten. Geeignete Lösungsmittel sind insbesondere aprotische bzw. wenig polare Lösungsmittel, z.B. Dimethoxymethan, Dimethoxyäthan, Diäthyläther oder Tetrahydrofuran.

Die Reaktionstemperatur liegt zweckmässigerweise zwischen ungefähr —20° und +50°C, vorzugsweise zwischen 0° und 20°. Tiefere Temperaturen sind industriell unpraktisch, höhere Temperaturen könnten leicht zu einer unerwünschten zweifachen Prenylierung des cis-4-Caranons führen.

Nach dem erfindungsgemässen Verfahren fällt I als Isomerengemisch von Ia und Ib an.

Die Trennung der Isomerengemische [in denen Ia überwiegt], kann, falls erwünscht, auf übliche Weise, z.B. mittels Säulenchromatographie oder präparativer Gaschromatographie erfolgen. Wie weiter unten ersichtlich, unterscheiden sich die Isomeren von I in ihren organoleptischen Eigenschaften nicht grundlegend, sodass aus wirtschaftlichen Gründen insbesondere das Isomerengemisch verwendet werden kann.

Die Verbindungen I weisen besondere organoleptische Eigenschaften auf, auf Grund derer sie sich vorzüglich als Riechstoffe eignen.

Die Erfindung betrifft demgemäss auch die Verwendung der Verbindungen I als Riechstoffe.

Die Verbindungen I, insbesondere Ia, zeichnen sich durch eine interessante Grün-Note aus, die bis jetzt in der Palette der Parfümerie fehlte, indem sie zugleich Facetten der blumigen, fettigen, krautigen und holzigen Richtung in einer Verbindung vereint enthält und ausserdem eine langhaftende Wirkung in Kompositionen entfaltet. Die Evaluation der olfaktorischen Eigenschaften der aus dem Gemisch von I isolierten Einzelkomponenten ergibt, dass die geruchliche Eigenart des erfindungsgemässen Ketongemisches I vorwiegend auf die Verbindung Ia zurückzuführen ist, sich aber die Isomeren der Formel Ib in ihren Geruchsnoten nicht wesentlich von Ia unterscheiden und insbesondere ohne jeden störenden Effekt auf den Gesamtgeruch sind.

Eine Auftrennung des Isomerengemisches ist daher, wie oben erwähnt, unwirtschaftlich, und das erfindungsgemäss erhältliche Gemisch Ia/Ib lässt sich mit Vorteil als solches zur Herstellung von Riechstoffkompositionen einsetzen.

So verbindet sich I mit zahlreichen bekannten Riechstoffingredienzen natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie aus der folgenden Zusammenstellung ersichtlich:

—*Naturprodukte*, wie Angelikasamenöl, Baummoos-Absolue, Bergamottöl, Cardamomenöl, acetyliertes Cedernholzöl (z.B. Vertofix R IFF bzw. Cedartone R Givaudan), Eichenmoos, Fichtennadelöl, Galbanumöl, Geraniumöl, Jasmin Absolue und seine Substitute, Lavendelöl, Lavandinöl, Patchouliöl, Petit-grainöl Paraguay, Sandelholzöl, Vetiveröl, Ylang-Ylang-Oel, Zitronenöl.

—*Alkohole*, wie cis-6-Nonenol, Linalool, Citronellol, Geraniol, natürliches Rhodinol, $\alpha$-Terpineol, Phenyläthylalkohol, Phenylpropylalkohol, Zimtalkohol.

—*Aldehyde*, wie 2,6-Dimethyl-5-heptenal, Decanal, Methylnonylacetaldehyd, Hydroxycitronellal, $\alpha$-Amylzimtaldehyd, Cyclamenaldehyd, p-tert. Butyl-$\alpha$-methyl-dihydro-zimtaldehyd (z.B. Lilial R Givaudan).

—*Ketone*, wie $\alpha$-Jonon, Acetylcedren, p-Methylacetophenon.

—*Ester*, wie cis-3-Hexenylacetat, cis-3-Hexenylbenzoat, Aethylacetoacetat, Linalylacetat, Geranylacetat, Terpenylacetat, Phenyläthylacetat, Styrallylacetat, p-tert. Butylcyclohexylacetat, 4[4-Methyl-3-pentenyl-]-cyclohex-3-en-1-yl-carbinylacetat (z.B. Myraldylacetat R Givaudan), Cinnamylformiat, Benzylacetat, Benzylsalicylat, Amylsalicylat.

—*Lactone*, wie $\gamma$-Undecalacton, Cumarin.

—*verschiedene weitere, in der Parfümerie oft benützten Komponenten* wie Moschus-Verbindungen (Ambrette-, Ketonmoschus, 12-Oxa-hexadecanolid (z.B. Musk 174 R Naarden), Indol, p-Menthan-8-thiol-3-on, Eugenol, Acetaldehydpropylphenyläthylacetal, Methyl-1-methylcyclododecyläther (z.B. Madrox R Givaudan).

Als besonders überraschend und wertvoll hat sich der durch den Zusatz von I erzielbare Effekt von Frische und Natürlichkeit in den entsprechenden Kompositionen, speziell der blumigen Richtungen, herausgestellt. Dieser Effekt war aufgrund des Geruchsablaufs der Einzelverbindung [konstanter Verlauf über 48 Stunden, insbesondere grün-holzig, aber ohne Frischeeffekt] nicht zu erwarten. Bei einer Substanz mit 15 C-Atomen würde man viel eher mit dem Gegenteil rechnen, nämlich einer gewissen dauerhaften Schwere.

Weiter überraschend ist die Tatsache, dass I in fruchtig-grünen Noten, wie im untenstehenden Beispiel 5 (Melone) gezeigt, trotz des eigenen Grün-Charakters nicht diese grüne Richtung unterstreicht, sondern eine das Fruchtfleisch mit seiner Süsse verstärkt wiedergebende Nuancierung hervortreten lässt.

Die Verbindungen der Formel I lassen sich daher in weiten Grenzen einsetzen, die beispielsweise von 0,1 (Detergentien) — 30% (alkoholische Lösungen) in Kompositionen reichen können, ohne das diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,5 und 25%. Die mit I hergestellten Kompositionen lassen sich für alle Arten von parfümierten Verbrauchsgütern einsetzen

(Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.). Insbesondere zeitigt die Verwendung von Kompositionen mit einem Gehalt an I in Seifen und Detergentien einen weiteren sehr erwünschten Effekt. So wird durch den Zusatz von I in der Komposition des untenstehenden Beispiels 3 der Geruch von auf diese Weise parfümierter Seife (1,2%) trotz der Zugabe der holzig-grün-fettigen Substanz I als sehr viel frischer und blumiger empfunden. Diese Note erhält sich auch über einen längeren Zeitraum.

Ein Waschpulver folgender Zusammensetzung:

| | |
|---|---|
| anionaktive Waschsubstanz | 6% |
| nichtionische Waschsubstanz | 5% |
| Seifenpulver | 7% |
| Natriumtripolyphosphat | 38% |
| Natriumsilikat | 7% |
| Carboxymethylcellulose | 1% |
| Wasser | 3% |
| Natriumsulfat | 33% |

wirkte nach Zugabe von beispielsweise 0,1—0,5%, insbesondere 0,2% der Riechstoffkomposition des Beispiels 3 ebenfalls sehr viel kräftiger in seiner blumig-grünen Duftnote. Damit vorgenommene Waschversuche zeigten im Vergleich zu dem ohne Zusatz von I parfümierten Waschpulver ein überraschendes Resultat: sowohl bei der Handwäsche (30°C) als auch bei der maschinellen Wäsche (60°C) zeigte sich die Ueberlegenheit der mit Zugabe von I versehenen Komposition, indem sich diese den damit gewaschenen Textilien verstärkt mitteilte. Die Verbindungen der Formel I zeichnen sich demgemäss überraschenderweise nicht nur durch rein parfümistische Effekte, sondern auch durch die Fähigkeit aus, selbst substantiv zu wirken und auch in Kombination mit anderen Riechstoffen diesen Effekt beizubehalten und damit die entsprechenden Riechstoffgemische substantiver wirken zu lassen.

## Beispiel 1

In einem Dreihalskolben mit Rührer, Tropftrichter und Thermometer werden 120 g (0,79 Mol) cis-4-Caranon vorgelegt und auf 0°C abgekühlt. Unter Rühren werden 110 g (1,67 Mol) gepulvertes 85%iges Aetzkali so zugegeben, dass die Temperatur 35°C nicht übersteigt. Nach beendeter Zugabe wird noch eine Stunde bei 0°C weiter gerührt. Dann werden 84 g (=0,8 Mol) Prenylchorid so zugetropft, dass die Temperatur wiederum höchstens auf 35°C steigt. Nach 12-stündigem Rühren bei Raumtemperatur wird das Reaktionsgemisch auf Eis gegossen, mit Diäthyläther erschöpfend extrahiert, die vereinigten Aetherextrakte mit Wasser neutral gewaschen, über Natriumsulfat getrocknet und anschliessend vom Lösungsmittel befreit. Das rohe Reaktionsprodukt wird über eine 50 cm- Füllkörperkolonne im Vakuum (Oelpumpe) fraktioniert destilliert und liefert so 60,5 g olfaktisch gutes Material von Siedepunkt 93°C/0,266 mbar $n_D^{20}$ 1,4841. Im Gaschromatogramm ist ein Hauptprodukt sowie ein vor und ein nach diesem eluiertes Nebenprodukt zu erkennen. Das Produktgemisch zeigt die ungefähre Zusammensetzung 11% (A), 83% (B), 6% (C).

Die Isomeren lassen sich mit Hilfe der präparativen Gaschromatographie in praktisch reiner (>95%) Form gewinnen und zeigen folgende spektrale Eigenschaften:

*A*: IR: 1700, 1452, 1376, 1205—1235 (breit), 1158, 1110, 1082, 1048, 1012, 986, 962, 830 $cm^{-1}$.

NMR: 0,64 (m, 1H); 0,92 (m, 1H); 0,96 (s, 3H); 0,98 (d, J=7, 3H); 1,06 (s, 3H); 1,20 (m, 1H); 1,62 + 1,73 (je s, je 3H); 2,0—2,5 (m, 5H); 5,1 (m, 1H) $\delta$ ppm.

MS: 220(11,M+), 151(12), 137(9), 123(18), 109(21), 95(28), 91(26), 79(27), 77(26), 69(30), 67(29), 55(29), 53(30), 41(100).

*B*: IR: 1698, 1452, 1408, 1376, 1348, 1292, 1205—1232 (breit) 1156, 1132, 1120, 1108, 1086, 1048, 1014, 990, 964, 896, 858 $cm^{-1}$.

NMR: 0,63—1,2 (m unter s, 2H); 0,88 (s, 3H); 0,91 (s, 3H); 1,07 (s, 3H); 1,68 + 1,73 (je s, 6H); 2,03 (m, 1H); 2,28—2,52 (m, 4H); 5,02 (t, J=7, 1H) $\delta$ ppm.

MS: 220(8,M+), 205(9), 151(25), 137(26), 123(100), 109(78), 95(32), 81(37), 69(56), 55(20), 53(19), 41(68).

C: IR: 1670, 1448, 1378, 1354, 1322, 1282, 1205—1235 (breit), 1116, 1082, 1040, 1012, 980, 910, 880 $cm^{-1}$.

NMR: 0,84—1,24 (m unter d und s, 2H); 0,95 (d, J=6, 3H); 1,16 (s, 6H); 1,36—1,53 (m, 2H); 1,66 (2 zusammenfallende s, 6H); 1,78—2,52 (m, 4H); 5,00 (t, J=6, 1H) $\delta$ ppm.

MS: 220(11,M+), 205(7), 152(47), 137(54), 123(24), 109(98), 95(74), 82(31), 69(100), 55(33), 53(29), 41(92).

Die Hauptkomponente B wird durch das $^{13}C$-NMR-Spektrum anhand der Singuletts bei 19,3 ppm (wie im cis-4-Caranon) und 46,12 ppm (neu) eindeutig als 3,7,7-Trimethyl-3-[3'-methyl-2'-butenyl]-bicyclo[4.1.0]-heptan-4-on identifiziert.

Die beiden Nebenkomponenten A und C weisen dagegen bereits im $^{1}H$-NMR-Spektrum das charakteristische Dublett der sekundären Methylgruppe auf, wie dieses auch im cis-4-Caranon beobachtet wird und stellen damit die isomeren 3,7,7,-Trimethyl-5-[3'-methyl-2'-butenyl-]-bicyclo[4.1.0]-heptan-4-one dar.

## Beispiel 2

In einem 200 ml Vierhalskolben mit Rührer, zwei Tropftrichtern, Thermometer und Rückflusskühler werden 30 ml Dimethoxymethan, die zuvor über basisches Aluminiumoxid gereinigt wurden, vorgelegt. Unter Rühren werden anschliessend 15 g (0,23 Mol) gepulvertes 85%iges Aetzkali eingetragen und der gesamte Kolbeninhalt wird auf 0°C abgekühlt. Bei dieser Temperatur werden hierauf gleichzeitig aus zwei Tropftrichtern 11,4 g (0,075 Mol) cis-4-Caranon und 7,8 g (0,0746 Mol) Prenylchlorid innerhalb 2 Stunden so zugetropft, dass die Temperatur + 2°C nicht übersteigt. Das Reaktionsgemisch wird noch 2 Stunden bei Raumtemperatur gerührt, dann auf Eis gegossen und mit Diäthyläther erschöpfend extrahiert. Die organische Phase wird mit Wasser neutral gewaschen, mit Natriumsulfat getrocknet und das Lösungsmittelgemisch am Rotationsverdampfer bei 30°C/26,66 mbar entfernt. Das Rohprodukt (14,6 g) wird über eine kurze Widmer-Kolonne im Hochvakuum fraktioniert destilliert. Nach einem Vorlauf von 4,1 g cis-4-Caranon kann I (7,6 g) bei 77°C/0,067 mbar destilliert werden ($n_D^{20}$ 1,4865). Es handelt sich um ein Isomerengemisch von 85% Ia (B des Beispiels 1) und 15% Ib (A des Beispiels 1).

In den folgenden Formulierungsbeispielen wird unter "I" das in den Beispielen 1 und 2 anfallende Isomerengemisch von Ia/Ib verstanden.

## Beispiel 3
### Blumige Base

| | Gewichtsteile |
|---|---|
| Propylenglykol | 200 |
| Benzylacetat | 100 |
| $\alpha$-Jonon | 100 |
| $\alpha$-Amylzimtaldehyd-Substitut | 100 |
| Citronellol | 50 |
| p-tert. Butylcyclohexylacetat | 50 |
| Linalool | 50 |
| Methyl-1-methylcyclododecyläther | 50 |
| Benzylsalicylat | 30 |
| Vertofix ® (IFF) | 30 |
| Eugenol | 30 |

| | Gewichtsteile |
|---|---|
| Cedartone R (Acetylcedren) | 30 |
| Styrallylacetat | 20 |
| Terpenylacetat | 20 |
| Musc 174 R (Naarden) | 20 |
| Geraniol | 20 |
| Linalylacetat | 20 |
| Ylang-Ylang-Oel | 10 |
| Acetaldehyd-propyl-phenyläthylacetal | 10 |
| Hydroxycitronellal | 5 |
| Methylnonylacetaldehyd (10% in Aethylphthalat) | 5 |
| | 950 |

Gibt man zu dieser blumigen Komposition, Richtung Hyacinthe, 50 Teile I, wird diese viel abgerundeter, weicher, blumiger und frischer. Die Jonon-Note wird sehr angenehm eingekleidet. Im Fond wird aus der Hyacinthe überraschenderweise eine Freesie. Die in der ursprüglichen Base pudrig-holzig erscheinende Note wird durch die blumige Note überdeckt. Die frische Note hält selbst über einen Zeitraum von 24 Stunden an.

Beispiel 4
Blumige Komposition Richtung Flieder

| | Gewichtsteile |
|---|---|
| Terpineol | 260 |
| Hydroxycitronellal | 200 |
| Phenyläthylalkohol | 160 |
| Zimtalkohol-Substitut | 100 |
| Phenylpropylalkohol | 100 |
| Cinnamylformiat | 20 |
| Geranylacetat | 10 |
| Keton-Moschus | 10 |
| Jasmin-Substitut | 10 |
| Eugenol | 5 |
| Indol (10% in Aethylalkohol) | 5 |
| p-Menthan-8-thiol-3-on (10% in Propylenglykol) | 5 |
| p-Methylacetophenon | 5 |

| | Gewichtsteile |
|---|---|
| C-10 Aldehyd (10% in Propylenglykol) | 5 |
| δ-Undelacton | 5 |
| | 900 |

Diese Base, ausschliesslich aus synthetischen Substanzen hergestellt, hinterlässt den Eindruck eines noch sehr synthetisch-chemisch wirkenden Flieders. Durch den Zusatz von 100 Teilen I erhält diese Base eine ungemein natürliche Note und erinnert nunmehr an frischen blühenden Flieder.

### Beispiel 5
### Fruchtige Parfümerie-Base Richtung Melone

| | Gewichtsteile |
|---|---|
| Propylenglykol | 180 |
| Linalylacetat | 120 |
| Myraldylacetat R (Givaudan) | 120 |
| Hexenylbenzoat | 100 |
| Cyclamenaldehyd | 80 |
| 2,6-Dimethyl-5-heptenal (10% in Aethylalkohol) | 60 |
| Aethylacetoacetat | 60 |
| cis-6-Nonenol (10% in Aethylalkohol) | 40 |
| Lilial R (Givaudan) | 20 |
| Hexenylacetat (10% in Alkohol) | 20 |
| | 800 |

Gibt man zu dieser fruchtigen Base 200 Teile I, so resultiert aus der ursprünglich fruchtig-grünen Melone mit einem an Melonenschale erinnernden Grundton eine viel süssere und abgerundete Melonennote. Die Fruchtfleisch-Note wird angenehm unterstrichen.

### Beispiel 6
### Chypre-Base Richtung Herren-Cologne

| | Gewichtsteile |
|---|---|
| Propylenglykol | 200 |
| Bergamotteöl | 100 |
| Methyl-1-methylcyclododecyläther | 100 |
| Hydroxycitronellal | 80 |
| Musc 174 R (Naarden) | 60 |
| Patchouliöl | 50 |
| Fichtennadelöl | 50 |

| | Gewichtsteile |
|---|---|
| Citronellol | 40 |
| Baummoos Absolue (50% in Propylenglykol) | 30 |
| Galbanumöl | 30 |
| Zitronenöl | 20 |
| Petitgrainöl Paraguay | 20 |
| Cedartone R (IFF) | 20 |
| Cardamomenöl | 5 |
| Angelikasamenöl | 5 |
| $\alpha$-Jonon | 40 |
| Linalool | 50 |
| | 900 |

Gibt man zu dieser Base, welche für Herren-Cologne-Richtungen verwendbar ist, 100 Teile I, so wirkt die so erhaltene Base viel leichter, blumiger und weicher. Die Substanz kleidet die harte, hervortretende Galbanum-Note der ursprünglichen Komposition sehr vorteilhaft ein und bildet zusammen mit der Jonon-Moschus-Note einen neuartigen Komplex.

Beispiel 7

Parfümerie-Komposition Richtung Fougère

| | Gewichtsteile |
|---|---|
| Lavendelöl | 200 |
| Amylsalicylat | 200 |
| Coumarin | 100 |
| Eichenmoos Absolue jugoslawisch | 80 |
| Geraniumöl Bourbon | 60 |
| Bergamotteöl | 60 |
| Ambrette Moschus | 60 |
| Petitgrainöl Paraguay | 40 |
| $\alpha$-Jonon | 40 |
| Vetiveröl Bourbon | 40 |
| Sandelholzöl | 40 |
| Patchouliöl | 20 |
| $\alpha$-Amylzimtaldehyd-Substitut | 20 |
| Eugenol | 20 |
| | 980 |

Gibt man zu dieser Fougère-Base 20 Teile I, so tritt in der neuen Komposition die Eichenmoos-Note zurück. Der Zusatz verleiht der neuen Base mehr Weichheit. Zudem wird die Lavendel-Note sehr angenehm unterstrichen, was einen wesentlich frischeren Eindruck der resultierenden Komposition zur Folge hat.

Beispiel 8
Parfümerie-Base Richtung Rose

| | Gewichtsteile |
|---|---|
| Phenyläthylalkohol | 400 |
| Citronellol | 180 |
| Geraniol | 150 |
| Linalool | 100 |
| $\alpha$-Jonon | 50 |
| Hydroxycitronellal | 50 |
| Phenyläthylacetat | 30 |
| Benzylacetat | 20 |
| | 980 |

Gibt man zu dieser konventionellen Rosen-Base 20 Teile I, so wird der rosige Charakter dieser Base wesentlich verändert: entspricht die ursprüngliche Base einer roten Rose, so wird mit dem Zusatz eine gelbe Rose erzeugt.

Beispiel 9
Parfümerie-Base Richtung Maiglöckchen

| | Gewichtsteile |
|---|---|
| Hydroxycitronellal | 430 |
| Rhodinol extra | 350 |
| Linalool | 80 |
| $\alpha$-Amylzimtaldehyd | 50 |
| Sandelholzöl | 50 |
| Ylang-Ylang-Oel | 20 |
| | 980 |

Gibt man zu dieser Maiglöckchen-Base 20 Teile I, so wird durch diesen Zusatz der typische frischgrüne Charakter dieser Blume hervorgehoben. Die Substanz verleiht der Base viel mehr Natürlichkeit.

**Patentansprüche**

1. Caranderivate der Formel

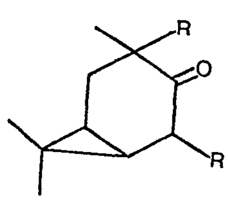

I

worin der eine Rest R für 3-Methyl-2-butenyl und der andere für Wasserstoff steht.

2. 3,7,7-Trimethyl-3-[3′-methyl-2′-butenyl]-bicyclo[4.1.0]-heptan-4-on.
3. 3,7,7-Trimethyl-5-[3′-methyl-2′-butenyl-]-bicyclo[4.1.0]-heptan-4-on.
4. Caranderivate der Formel

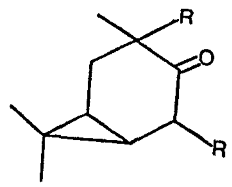

I

worin der eine Rest R für 3-Methyl-2-butenyl und der andere für Wasserstoff steht, als Riechstoffe.

5. Riechstoffkomposition, gekennzeichnet durch einen Gehalt an einen Caranderivat der Formel

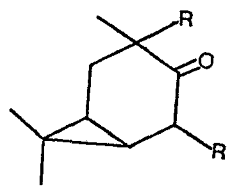

I

worin der eine Rest R für 3-Methyl-2-butenyl und der andere für Wasserstoff steht.

6. Verfahren zur Herstellung von Caranderivaten der Formel

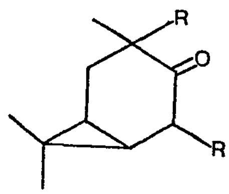

I

worin der eine Rest R für 3-Methyl-2-butenyl und der andere für Wasserstoff steht, dadurch gekennzeichnet, dass man 4-Caranon mit einem 3-Methyl-2-butenylhalogenid umsetzt.

7. Verfahren nach Anspruch 6, dadurch gekennzeichnet, dass man cis-4-Caranon als Ausgangsmaterial verwendet.

8. Verwendung von Caranderivaten der Formel

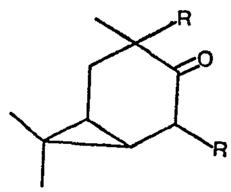

I

worin der eine Rest R für 3-Methyl-2-butenyl und der andere für Wasserstoff steht, als Riechstoffe.

## Claims

1. Carane derivates of the formula

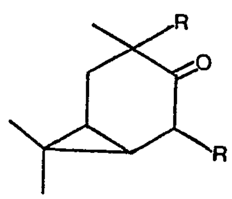

I

wherein one of the R radicals represents 3-methyl-2-butenyl and the other represents hydrogen.

2. 3,7,7-Trimethyl-3-(3′-methyl-2′-butenyl)-bicyclo-[4.1.0]-heptan-4-one.
3. 3,7,7-Trimethyl-5-(3′-methyl-2′-butenyl)-bicyclo-[4.1.0]-heptan-4-one.
4. Carane derivatives of the formula

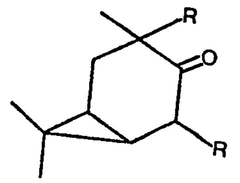

I

wherein one of the R radicals represents 3-methyl-2-butenyl and the other represents hydrogen, as odorant substances.

5. Odorant composition, characterized by a content of a carane derivate of the formula

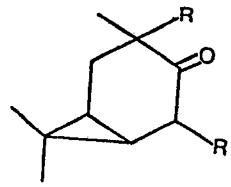

I

wherein one of the R radicals represents 3-methyl-2-butenyl and the other represents hydrogen.

6. Process for the manufacture of carane derivatives of the formula

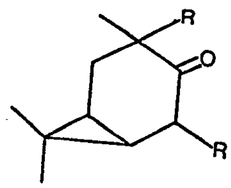

I

wherein one of the R radicals represents 3-methyl-2-butenyl and the other represents hydrogen, characterized in that one reacts 4-caranone with a 3-methyl-2-butenyl-halogenide.

7. A process according to claim 6, wherein cis-4-caranone is used as the starting material.

8. The use of carane derivatives of the formula

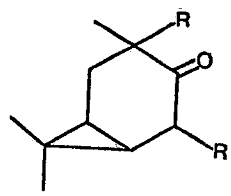

I

wherein one of the R radicals represents 3-methyl-2-butenyl and the other represents hydrogen, as odorants.

**Revendications**

1. Dérivés de carane, de formule

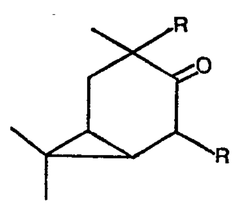

I

dans laquelle l'un des symboles R représente le groupe 3-méthyl-2-butényle et l'autre un atome d'hydrogène.

2. La 3,7,7-triméthyl-3-[3′-méthyl-2′-butény!]-bicyclo[4.1.0]-heptane-4-one.

3. La 3,7,7-triméthyl-5-[3′-méthyl-2′-butényl]-bicyclo[4.1.0]-heptane-4-one.

4. Dérivés de carane, de formule

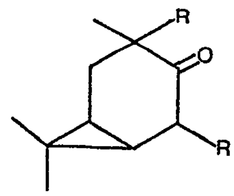

I

dans laquelle l'un des symboles R représente le groupe 3-méthyl-2-buténylе et l'autre l'hydrogène, en tant que substances odorantes.

5. Composition odorante caractérisée en ce qu'elle contient un dérivé de carane de formule

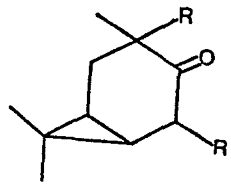

I

dans laquelle l'un des symboles R représente le groupe 3-méthyl-2-buténylе et l'autre l'hydrogène.

6. Procédé de préparation des dérivés de carane de formule

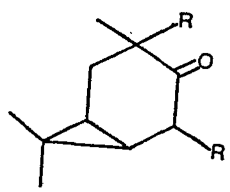

I

dans laquelle l'un des symboles R représente le groupe 3-méthyl-2-buténylе et l'autre l'hydrogène, caractérisé en ce que l'on fait réagir la 4-caranone avec un halogénure de 3-méthyl-2-buténylе.

7. Procédé selon la revendication 6, caractérisé en ce que l'on utilise comme produit de départ la cis-4-caranone.

8. Utilisation des dérivés de carane de formule

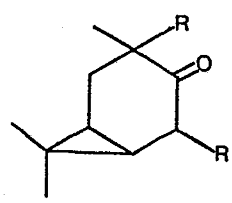

I

dans laquelle l'un des symboles R représente le groupe 3-méthyl-2-buténylе et l'autre l'hydrogène, en tant que substances odorantes.